Europäisches Patentamt

European Patent Office

Office européen des brevets

(19)

(11) Numéro de publication: **0 392 978**
A1

# DEMANDE DE BREVET EUROPEEN

(12)

(21) Numéro de dépôt: 90810272.6

(22) Date de dépôt: 05.04.90

(51) Int. Cl.5: **F04B 43/04, A61M 5/142**

(30) Priorité: 11.04.89 CH 1369/89

(43) Date de publication de la demande:
17.10.90 Bulletin 90/42

(84) Etats contractants désignés:
AT BE CH DE DK ES FR GB GR IT LI LU NL SE

(71) Demandeur: WESTONBRIDGE
INTERNATIONAL LIMITED
43, 45 Northumberland road

Ballsbridge, Dublin 4(IE)

(72) Inventeur: van Lintel, Harald T. G.
Voortsweg 20
NL-7523 CH Enschede(NL)

(74) Mandataire: Lamoureux, Bernard Jean et al
ICB, Ingénieurs Conseils en Brevets S.A.,
Passage Max Meuron 6
CH-2001 Neuchâtel(CH)

(54) **Micropompe à débit constant.**

(57) Micropompe comprenant une chambre de pompage (58), un canal d'entrée (70) communiquant avec la chambre de pompage par un clapet d'aspiration (62, 64) et un canal de sortie (72a, 72b) communiquant avec la chambre de pompage par un clapet de refoulement (66, 68), ces éléments étant réalisés par gravure d'une plaquette en silicium (52) qui est ensuite accolée de manière étanche à des plaquettes en verre (54, 56), la micropompe comprenant en outre une pastille piézoélectrique (80) pour faire varier le volume de la chambre de pompage par déformation d'une paroi (78) formant une partie de la paroi de cette chambre de pompage.

Selon l'invention, la chambre de pompage comporte une butée (60) qui détermine l'amplitude du mouvement de la paroi déformable (78). Ainsi, la variation de volume de la chambre causée par le déplacement de la paroi déformable est parfaitement défini, ce qui permet de rendre constant le débit de la micropompe dans les conditions normales d'utilisation.

Fig.7A

EP 0 392 978 A1

## MICROPOMPE A DEBIT CONSTANT

La présente invention concerne une micropompe du type dans lequel une partie au moins du mécanisme de la pompe est réalisée par usinage d'une plaquette en silicium à l'aide des techniques de photolithographie.

Les micropompes peuvent être utilisées notamment pour l'administration in situ de médicaments, la miniaturisation de la pompe permettant éventuellement une implantation permanente de celle-ci dans le corps. Ces pompes permettent un dosage précis de faibles quantités de fluide à injecter.

De telles micropompes sont décrites notamment dans l'article "A piezoelectric micropump based on micromachining of silicon" de H. van Lintel et al. paru dans Sensors and Actuators, no 15, 1988, pp 153-157. Ces micropompes comportent essentiellement un empilement de trois plaquettes, c'est-à-dire une plaquette en silicium disposée entre deux plaquettes en verre.

La plaquette en silicium est gravée pour former une cavité, qui avec l'une des plaquettes en verre définit la chambre de pompage, au moins un clapet d'aspiration et au moins un clapet de refoulement mettant la chambre de pompage en communication respectivement avec un canal d'entrée et un canal de sortie. La partie de la plaquette en verre formant une paroi de la chambre de pompage peut être déformée par un élément de commande constitué par exemple par une pastille piézoélectrique. Celle-ci est équipée de deux électrodes qui, lorsqu'elles sont raccordées à une source de tension électrique, provoquent la déformation de la pastille et, par suite, la déformation de la plaquette en verre, ce qui provoque une variation du volume de la chambre de pompage. La paroi déformable de la chambre de pompage peut ainsi être déplacée entre une première position, dans laquelle elle est relativement éloignée de la paroi opposée, lorsque la pastille piézoélectrique n'est soumise à aucune tension électrique, et une seconde position, dans laquelle elle est plus proche de la paroi opposée, lorsqu'une tension est appliquée entre les électrodes de la pastille piézoélectrique.

Le fonctionnement de la micropompe est le suivant. Lorsque aucune tension électrique n'est appliquée à la pastille piézoélectrique, les clapets d'aspiration et de refoulement sont en position fermée. Lorsqu'une tension électrique est appliquée, il se produit une augmentation de pression dans la chambre de pompage qui provoque l'ouverture du clapet de refoulement dès que la pression dans la chambre est supérieure à la somme de la pression dans le canal de sortie et de la pression créée par la pré-tension du clapet. Le fluide contenu dans la chambre de pompage est alors refoulé vers le canal de sortie par le déplacement de la paroi déformable de la première position vers la seconde position. Pendant cette phase, le clapet d'aspiration est maintenu fermé par la pression régnant dans la chambre de pompage.

Au contraire, lorsque l'on fait décroître la tension électrique, la pression dans la chambre de pompage diminue. Ceci provoque la fermeture du clapet de refoulement, dès que la pression dans la chambre de pompage est inférieure à la somme de la pression dans le canal de sortie et de la pression créée par la pré-tension du clapet, et l'ouverture du clapet d'aspiration, dès que la somme de la pression dans la chambre de pompage et de la pression créée par la pré-tension du clapet est inférieure à la pression dans le canal d'entrée. Il y a alors aspiration de fluide dans la chambre de pompage par le canal d'entrée par suite du déplacement de la paroi déformable de la seconde position vers la première position.

Comme on l'a déjà indiqué, ces micropompes sont utilisées notamment pour l'administration de médicaments. Il est donc important que le débit de la micropompe soit bien déterminé, de manière que le médicament à injecter soit dosé de manière très précise. Or, les micropompes connues présentent sur ce point certaines imperfections.

En effet, le débit de la micropompe dépend de la variation de volume de la chambre de pompage entre les deux positions de la paroi déformable. Cette variation de volume dépend de plusieurs paramètres, parmi lesquels la tension électrique appliquée à la pastille piézoélectrique et les caractéristiques physiques de la pastille piézoélectrique (épaisseur, diamètre, constante diélectrique) et de la paroi déformable (matériau, épaisseur). Ainsi, une même tension électrique appliquée à des micropompes en apparence identiques pourra provoquer des déformations différentes des chambres de pompage de ces micropompes qui, par suite, présenteront des débits différents.

Par ailleurs, pour une même micropompe, le débit peut évoluer au cours du temps à cause du vieillissement des matériaux. Enfin, le débit de la micropompe dépend de la pression dans le canal de sortie, puisque le clapet de refoulement ne s'ouvre que lorsque la pression dans la chambre de pompage est supérieure à la somme de la pression dans le canal de sortie et de la pression créée par la pré-tension du clapet.

H. van Lintel et al. ont décrit dans l'article déjà cité une micropompe dotée d'un clapet supplémentaire qui permet de rendre le débit moins dépendant de la pression dans le canal de sortie. Cependant, cette micropompe ne permet pas de résoudre les autres inconvénients évoqués plus haut.

L'invention a pour but de résoudre notamment les inconvénients mentionnés, afin d'atteindre un débit sensiblement constant de la micropompe, et en particulier indépendant des tolérances de fabrication de la micropompe, du vieillissement de celle-ci et de la pression dans le canal de sortie.

La micropompe selon l'invention comprend classiquement une pluralité de plaquettes accolées l'une à l'autre de manière étanche dans laquelle sont formées une chambre de pompage définie par deux plaquettes accolées délimitant une cavité obtenue par gravure d'au moins l'une de ces plaquettes, au moins un clapet d'aspiration et au moins un clapet de refoulement mettant la chambre de pompage en communication respectivement avec un canal d'entrée et un canal de sortie, cette micropompe comprenant en outre un élément de commande pour déformer élastiquement la partie d'une plaquette constituant une paroi de la chambre de pompage entre une première position, où cette paroi déformée est plus éloignée de la paroi opposée de la chambre de pompage et une seconde position, où cette paroi est relativement proche de cette paroi opposée, les déplacements de la paroi déformable provoquant l'aspiration ou le refoulement d'un fluide. Selon l'invention, cette micropompe se caractérise en ce que la chambre de pompage comporte une butée qui détermine la seconde position de la paroi déformable.

Cette butée limite le mouvement de la paroi déformable vers la paroi opposée de la chambre de pompage. Ceci permet de définir de manière très précise le volume de la chambre de pompage à la fin de l'opération de refoulement du fluide.

Par ailleurs, grâce à cette butée, il n'est plus nécessaire que la tension électrique de commande de la pastille piézoélectrique, ou plus généralement l'intensité du signal appliqué à l'élément de commande de déformation de la paroi déformable, ait une valeur précise. Il suffit simplement que cette tension soit supérieure à celle qui est nécessaire pour que la butée entre en contact avec la paroi de la chambre de pompage qui lui est opposée.

Enfin, la butée permet d'avoir un débit sensiblement indépendant de la pression régnant dans le canal de sortie puisqu'il est possible d'imposer à la pastille piézoélectrique une tension élevée, induisant une pression élevée dans la chambre de pompage, donc supérieure à la somme de la pression régnant dans le canal de sortie dans les conditions normales d'utilisation et de la pression créée par la pré-tension du clapet de refoulement, sans que ceci se traduise par une augmentation de l'amplitude du mouvement de la paroi déformable, qui reste fixé par la butée.

Cette butée peut être réalisée notamment sous la forme d'un ou de plusieurs bossages, qui peuvent être formés dans le fond de la cavité lors de la gravure de la plaquette dans laquelle cette cavité est réalisée, et/ou réalisé par gravure, collage ou autre sur la paroi déformable. La butée peut également être constituée simplement par le fond même de cette cavité dès lors que la hauteur de la chambre de pompage est choisie égale à l'amplitude désirée du mouvement de la paroi déformable.

Les caractéristiques et avantages de l'invention ressortiront mieux de la description qui va suivre, donnée à titre illustratif mais non limitatif, en référence aux dessins annexés, sur lesquels :

- la figure 1A représente, une coupe suivant la ligne I-I d'une chambre de pompage d'une micropompe conforme à l'invention, dans laquelle la paroi déformable est représentée dans la première position,

- la figure 1B représente une vue de dessous de la chambre de pompage représentée sur la figure 1A,

- la figure 2 représente une coupe suivant la ligne I-I de la chambre de pompage des figures 1A et AB, dans laquelle la paroi déformable se trouve dans la seconde position,

- les figures 3A et 3B représentent respectivement une coupe suivant la ligne III-III et une vue de dessous d'une variante de réalisation d'une chambre de pompage pour une micropompe conforme à l'invention,

- la figure 4 est une coupe transversale d'une autre variante de réalisation d'une chambre de pompage pour une micropompe selon l'invention, dans laquelle la paroi déformable se trouve dans la première position,

- la figure 5 représente, en coupe transversale, la chambre de pompage de la figure 4 dans laquelle la paroi déformable occupe la seconde position,

- les figures 6A et 6B représentent respectivement une coupe suivant la ligne VI-VI et une vue de dessous d'une micropompe selon l'invention,

- les figures 7A et 7B représentent respectivement une coupe suivant la ligne VII-VII et une vue de dessus d'une autre micropompe selon l'invention, et

- la figure 8 est un diagramme illustrant le débit d'une micropompe en fonction de la pression dans le canal de sortie, pour une micropompe à deux clapets de type connu et pour une micropompe selon l'invention.

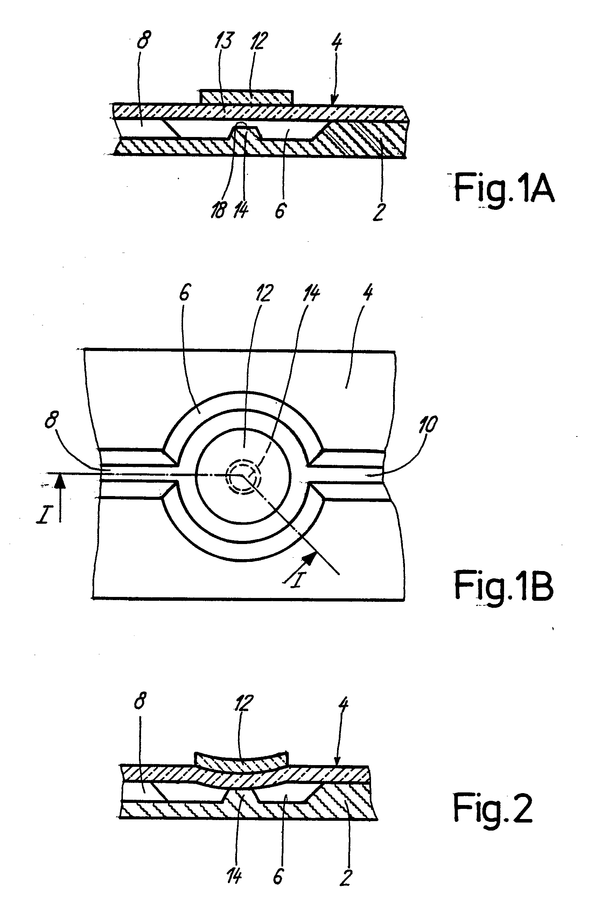

Un premier mode de réalisation d'une chambre de pompage pour une micropompe conforme à l'invention va être décrite en référence aux figures 1A, 1B, 1C. Cette chambre de pompage est délimitée par des plaquettes 2, 4 accolées l'une à l'autre de manière étanche, par exemple par soudage anodique ou par collage. Ces plaquettes ont généralement une épaisseur de l'ordre de quelques dixièmes de milimètre. La cavité 6 définissant la chambre de pompage, ainsi qu'un canal d'entrée 8 et un canal de sortie 10, sont obtenus par gravure de la plaquette 2 à l'aide des techniques de photolithographie bien connues, telle que la gravure en phase liquide. La cavité a un diamètre de l'ordre de 1 cm et une hauteur comprise entre 5 et 200 micromètres. La plaquette 2 est en un matériau qui peut être facilement gravé, tel que le silicium monocristallin; la plaquette 4 est par exemple en verre.

Un élément de commande tel que, par exemple, une pastille piézoélectrique 12 est collé sur la face extérieure de la plaquette 4, au niveau de la cavité 6. Cette pastille piézoélectrique est recouverte sur chacune de ses faces d'une électrode reliée à une source de tension (non représentée).

Les figures 1A et 2 illustrent respectivement la position de la plaquette 4 selon qu'aucune tension électrique n'est appliquée à la pastille piézoélectrique 12 (première position) ou qu'une tension électrique est appliquée à cette pastille piézoélectrique (seconde position).

Selon l'invention, la chambre de pompage est munie d'une butée 14 qui, en limitant l'amplitude du mouvement de la paroi déformable 13 de la plaquette 4) détermine parfaitement la seconde position de cette paroi déformable. Il en résulte que le volume de la chambre de pompage, à la fin de l'opération de refoulement, c'est-à-dire lorsque la paroi déformable 13 se trouve dans la seconde position, a une valeur parfaitement définie et reproductible.

La distance entre la butée et la paroi opposée de la chambre, lorsque la paroi déformable est dans la première position, est de l'ordre de 10 μm ou moins. Cette distance dépend évidemment des dimensions de la chambre de pompage et du débit de fluide désiré.

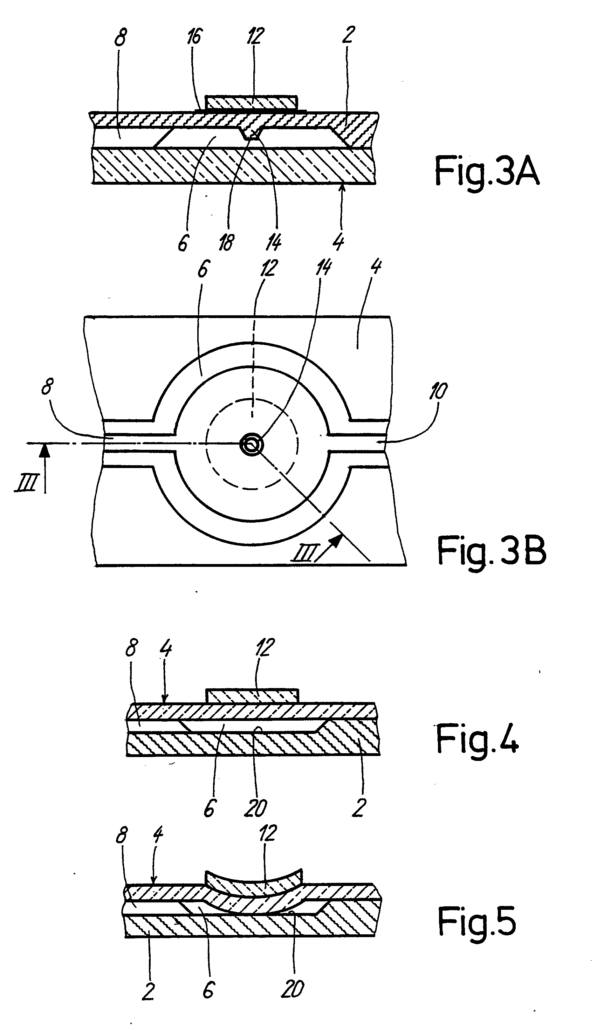

Dans le mode de réalisation représenté sur les figures 1A, 1B et 2, la pastille piézoélectrique 12 est fixée sur la plaquette en verre 4. Il est bien entendu possible de fixer la pastille piézoélectrique 12 sur la plaquette en silicium 2. Une telle chambre de pompage a été représentée en coupe suivant la ligne III-III et en vue de dessous respectivement sur les figures 3A et 3B.

Sur ces figures, les éléments identiques à ceux représentés sur la figures 1A, 1B et 2 portent les mêmes références. Dans le cas où la plaquette en silicium 2 porte la pastille piézoélectrique 12, une couche 16 de SiO₂ est interposée entre la plaquette 2 et la pastille piézoélectrique 12 pour assurer une isolation électrique. Enfin, il faut noter que, dans ce mode de réalisation, il est nécessaire que la butée 14 ait un diamètre nettement inférieur à celui de la pastille piézoélectrique, pour ne pas trop limiter la flexibilité de la plaquette 2.

Dans les deux premiers modes de réalisation décrits, la butée 14 est constituée par un bossage qui s'étend à partir d'une paroi de la chambre de pompage. Ce bossage est réalisé dans la plaquette en silicium 2, lors de la gravure de la cavité et des canaux d'entrée et de sortie. La surface supérieure 18 du bossage, contre laquelle vient buter la paroi opposée de la chambre de pompage lorsque la pastille piézoélectrique est soumise à une tension électrique, est de préférence plane. Ceci permet de définir de manière plus précise la seconde position de la paroi déformable.

Il est également possible d'utiliser comme butée le fond de la cavité lui-même. C'est le cas lorsque l'on réalise une cavité dont la hauteur est égale à l'amplitude du mouvement désiré de la paroi déformable. Les figures 4 et 5 représentent des coupes transversales d'une telle chambre de pompage respectivement dans la première position et dans la seconde position de la plaquette déformable 4. Sur ces figures, la chambre de pompage est définie par une cavité 6 reliée à un canal d'entrée 8 et un canal de sortie (non représenté). Cette chambre de pompage est composée d'une plaquette en silicium 2 et d'une plaquette en verre 4 comme dans les figures précédentes. La pastille piézoélectrique est disposée sur la plaquette en verre 4 ; il est bien entendu que cette pastille 12 peut également être disposée sur la plaquette en silicium 2, comme dans les figures 3A et 3B.

L'utilisation du fond 20 de la cavité 6 comme butée pour la paroi déformable présente l'avantage de réduire le nombre d'opérations nécessaires pour graver la plaquette en silicium 2, par rapport aux modes de réalisations précédents dans lesquels la butée est constituée par un bossage. De plus, comme le montre la figure 5, le volume de la chambre à la fin de la phase de refoulement est très faible. Ceci assure un pompage efficace même si le liquide contient beaucoup de bulles de gaz (à condition que le volume parasite entre les clapets et la chambre elle-même soient aussi très faibles). Au contraire, si le volume de la chambre de pompage reste assez important à la fin de la phase de refoulement, et c'est généralement le cas lorsque la butée est un bossage, les bulles de gaz peuvent être comprimées sans être expulsées de la chambre de pompage.

En revanche, il faut noter que la résistance à l'écoulement du fluide est plus grande avec une chambre de pompage telle que représentée sur la figure 4, qui convient donc surtout pour des micropompes à très faible débit.

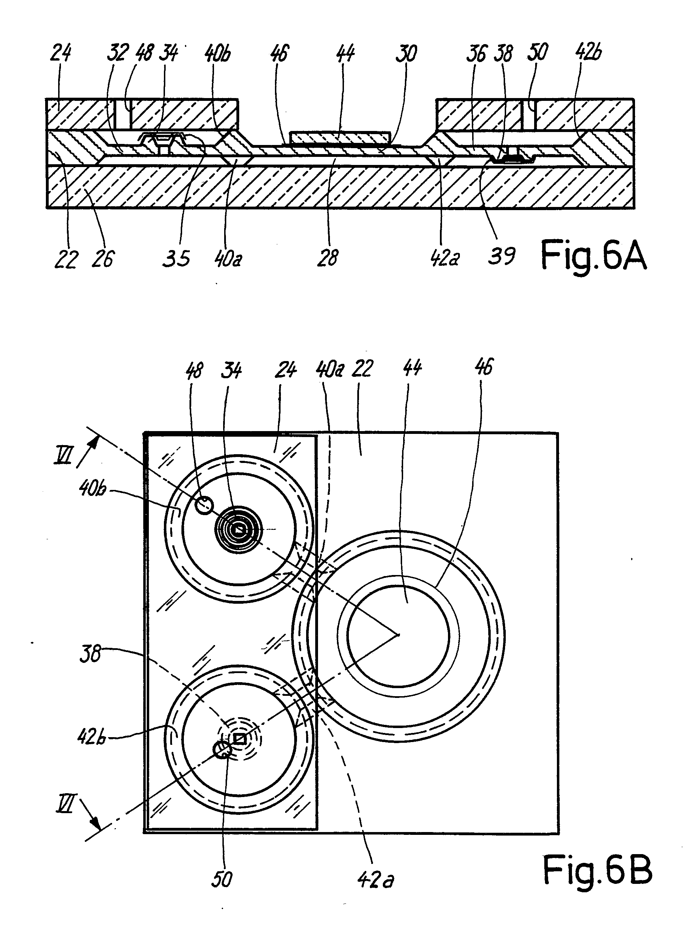

Un mode de réalisation d'une telle micropompe selon l'invention est représenté en coupe suivant la ligne VI-VI et en vue de dessous respectivement sur les figures 6A et 6B. Cette micropompe comprend principalement une plaquette en silicium 22 disposée entre des plaquettes en verre 24 et 26. La plaquette 22 est gravée sur une face pour former une cavité 28, définissant la chambre de pompage, et sur l'autre face pour régler l'épaisseur de la partie de la plaquette 22 qui constitue la paroi déformable 30 de la chambre de pompage. Cette épaisseur est par exemple de 150 $\mu$m.

Les deux faces de la plaquette 22 sont en outre gravées pour former une membrane 32 et une nervure annulaire 34 d'un clapet d'aspiration, une membrane 36 et une nervure annulaire 38 d'un clapet de refoulement, et un canal d'entrée 40a, 40b et un canal de sortie 42a, 42b. Pour éviter une éventuelle adhésion des clapets sur les plaquettes en verre, on recouvre ceux-ci d'une fine couche 35, 39 de SiO₂.

La pastille piézoélectrique 44 permettant de commander le mouvement de la paroi déformable 30 est collée à l'aide d'une colle cyano-acrylate après que la paroi déformable a été recouverte d'une fine couche 46 de SiO₂, pour assurer une isolation électrique. La pastille piézoélectrique 44 peut être du type PXE-5 de Philips, ayant un diamètre de 10 mm et une épaisseur de 0,20 mm.

La paroi déformable 30 et les membranes 32, 36 étant formées dans la plaquette en silicium 22, cette dernière est de préférence une plaquette de silicium monocristallin d'orientation <100>, qui présente de bonnes propriétés mécaniques et se prête bien à la gravure. Cette plaquette peut avoir un diamètre de 5 cm et une épaisseur de l'ordre de 300 micromètres.

·Les plaquettes 24 et 26 sont en verre poli. Elles ont un diamètre de 5 cm et une épaisseur de 1 mm. La plaquette 24 est percée d'un trou d'entrée 48 et d'un trou de sortie 50. Les plaquettes 24 et 26 sont accolées de manière étanche à la plaquette 22 au moyen de la technique connue sous le nom de soudage anodique.

Dans le mode de réalisation représenté sur les figures 6A et 6B, la hauteur de la chambre de pompage, c'est-à-dire la distance entre la paroi déformable 30 et la plaquette 26 lorsque aucune tension électrique n'est appliquée à la pastille piézoélectrique 44, est choisie (lors de la gravure de la plaquette 22) de sorte que la butée est formée par la surface de la plaquette 26. La chambre de pompage est donc similaire à celle décrite en référence aux figures 4 et 5, la seule différence étant que la pastille piézoélectrique est fixée sur la plaquette en silicium au lieu de l'être sur la plaquette en verre.

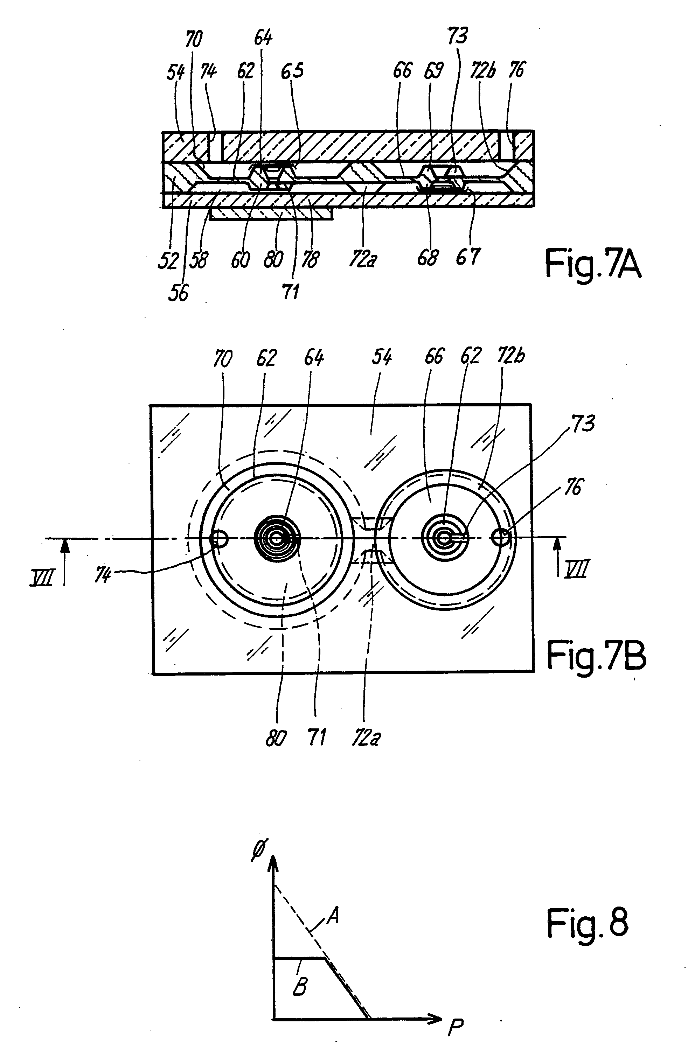

On a représenté respectivement sur les figures 7A et 7B, une coupe suivant la ligne VII-VII et une vue de dessus d'une micropompe selon un autre mode de réalisation de l'invention. Cette micropompe présente une plus grande compacité que la micropompe représentée sur les figures 6A et 6B. Ceci est obtenu en plaçant le clapet d'aspiration de la micropompe directement sur l'une des parois de la chambre de pompage. Il serait possible d'y placer en outre une partie du clapet de refoulement.

Cette micropompe se compose d'une plaquette en sicilicum 52 disposée entre deux plaquettes en verre 54 et 56. Une face de la plaquette 52 est gravée pour former une cavité 58, définissant la chambre de pompage et lors de cette opération de gravure, un bossage 60 est formé pour constituer une butée selon l'invention. Les deux faces de la plaquette en silicium 52 sont également gravées pour former une membrane 62 et une nervure annulaire 64 d'un clapet d'aspiration, une membrane 66 et une nervure annulaire 68 d'un clapet de refoulement, et un canal d'entrée 70 et un canal de sortie 72a, 72b. Des couches 65, 67 en SiO₂ sont formées sur les nervures annulaires 64, 68 pour éviter l'adhésion des clapets sur les plaquettes en verre.

Le clapet d'aspiration est de préférence centré sur la cavité 58. Dans ces conditions, le bossage 60, également centré par rapport à la cavité 58 et au clapet d'aspiration, se présente sous la forme d'un anneau. Les clapets peuvent être munis d'un limiteur d'amplitude pour réduire les risques de cassure de la membrane. Pour le clapet de refoulement, ce limiteur est constitué par une nervure annulaire 69; pour le clapet d'aspiration, c'est le bossage 60 qui jour le rôle de limiteur. Des canaux 71, 73 sont de préférence ménagés dans les limiteurs d'amplitude des clapets, pour permettre l'écoulement du fluide lorsque ces limiteurs sont en contact avec les plaquettes en verre 54, 56. ·

Après les opérations de gravure, les plaques en verre 54 et 56 sont fixées de manière étanche par soudage anodique sur la plaquette en silicium 52, la plaquette en verre 54 étant munie d'une ouverture d'entrée 74 et d'une ouverture de sortie 76. La paroi déformable 78 de la chambre de pompage est constituée par une partie de la plaquette en verre 56; son épaisseur est de l'ordre de 200 $\mu$m.

Une pastille piézoélectrique 80 est collée sur cette paroi 78 pour en commander le mouvement. Conformément à l'invention, le bossage annulaire 60, limite l'amplitude du mouvement de la paroi déformable, ce qui permet de définir de manière précise le volume de la chambre de pompage à la fin de l'opération de refoulement.

Cette butée permet aussi de rendre constant le débit de la micropompe dans les conditions normales d'utilisation. Comme on peut le voir sur le diagramme de la figure 8, le débit Ø d'une micropompe classique à deux clapets est une fonction linéaire de la pression p régnant à la sortie de la micropompe (courbe A). En revanche, le débit Ø d'une micropompe selon l'invention est sensiblement constant, dans les gammes des pressions normales d'utilisation (courbe B). Ceci résulte de ce que, pour une pression inférieure à une pression maximale d'utilisation, la variation de volume provoquée par le déplacement de la paroi déformable est limitée. Le débit est ainsi pratiquement le même que celui correspondant à la pression maximale d'utilisation.

## Revendications

1. Micropompe comprenant une pluralité de plaquettes (2, 4; 22, 24, 26 ; 52, 54, 56) accolées l'une à l'autre de manière étanche, dans laquelle sont formés une chambre de pompage (6 ; 28 ; 58) définie par deux plaquettes accolées délimitant une cavité formée par gravure d'au moins l'une de ces plaquettes, au moins un clapet d'aspiration ( 32, 34 ; 62, 64) et au moins un clapet de refoulement (36, 38 ; 66, 68) mettant la chambre de pompage en communication respectivement avec un canal d'entrée et un canal de sortie, ladite micropompe comprenant en outre un élément de commande (12, 44, 80) pour déformer élastiquement la partie d'une plaquette constituant une paroi (13 ; 30 ; 78) de la chambre de pompage entre une première position où ladite paroi déformable est relativement éloignée de la paroi opposée de la chambre de pompage et une seconde position où ladite paroi déformable est plus proche de ladite paroi opposée, les déplacements de la paroi déformable provoquant l'aspiration d'un fluide dans la chambre de pompage ou le refoulement de celui-ci, ladite micropompe étant caractérisée en ce que ladite chambre de pompage comporte une butée (14 ; 20 ; 60) qui détermine ladite seconde position de la paroi déformable.

2. Micropompe selon la revendication 1, caractérisée en ce que la butée est un bossage (14 ; 60) formé sur une face intérieure de la chambre de pompage (6 ; 58).

3. Micropompe selon la revendication 2, caractérisée en ce que la surface (18) de la butée qui entre en contact avec une face intérieure de la chambre de pompage, lorsque la paroi déformable occupe la seconde position, est plane.

4. Micropompe selon l'une quelconque des revendications 2 et 3, caractérisée en ce que le bossage (14) est formé dans le fond de la cavité, lors de la gravure de celle-ci.

5. Micropompe selon la revendication 1, caractérisée en ce que la butée est constituée par la face intérieure (20) de la paroi de la chambre de pompage située en regard de la paroi déformable (12).

6. Micropompe selon l'une quelconque des revendications 1 à 5, caractérisée en ce que les plaquettes définissant la chambre de pompage sont l'une (20 ; 22; 52) en silicium et l'autre (4 ; 26; 56) en verre, la cavité et la butée étant formées par gravure de la plaquette en silicium.

7. Micropompe selon la revendication 6, caractérisée en ce que la paroi déformable (13; 30) est une partie de la plaquette en silicium (2; 22).

8. Micropompe selon l'une quelconque des revendications 6 et 7, caractérisée en ce que la plaquette en silicium (2; 22) est en silicium monocristallin.

9. Micropompe selon l'une quelconque des revendications 1 à 8, caractérisée en ce qu'un clapet d'aspiration est disposé dans la paroi de la chambre de pompage opposée à la paroi déformable, et en ce qu'une partie (60) au moins de ce clapet constitue la butée.

10. Micropompe selon l'une quelconque des revendications 1 à 7, caractérisée en ce que l'élément de commande comprend une pastille piézoélectrique (12 ; 44 ; 76) fixée sur la paroi déformable.

Fig.1A

Fig.1B

Fig.2

Fig.3A

Fig.3B

Fig.4

Fig.5

Fig.6A

Fig.6B

Fig.7A

Fig.7B

Fig.8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,D | SENSORS & ACTUATORS, vol. 15, no. 2, octobre 1988, pages 153-167, Elsevier Sequoia, Lausanne, CH; H.T.G. VAN LINTEL et al.: "A piezoelectric micropump based on micromachining of silicon" * Pages 154,161,162 * --- | 1-10 | F 04 B   43/04 A 61 M    5/142 |
| Y | PATENT ABSTRACTS OF JAPAN, vol. 9, no. 326 (M-441)[2049], 21 décembre 1985; & JP-A-60 159 387 (SHARP K.K.) 20-08-1985 * Abrégé; figures * --- | 1-10 | |
| A | WO-A-8 707 218  (SIEMENS AG)(03-10-1987) --- | | |
| A | EP-A-0 134 614  (VITAFIN N.V.)(20-03-1985) ----- | | |

**DOMAINES TECHNIQUES RECHERCHES (Int. Cl.5)**

F 04 B
A 61 M

Le présent rapport a été établi pour toutes les revendications

| Lieu de la recherche | Date d'achèvement de la recherche | Examinateur |
|---|---|---|
| LA HAYE | 20-07-1990 | CLARKSON P.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P0402)